# EUROPEAN PATENT SPECIFICATION

(11) **EP 2 162 164 B1**
(45) Date of publication and mention of the grant of the patent: **11.05.2011**
(21) Application number: 08758310.0
(22) Date of filing: 08.07.2008
(51) Int. Cl.: A61M 3/02

(54) **A STRAP FOR SUPPORTING A LIQUID RESERVOIR**
SCHLAUFE ZUR HALTERUNG EINES FLÜSSIGKEITSBEHÄLTERS
SANGLE DE SUPPORT POUR RÉSERVOIR DE LIQUIDE

(30) Priority: 09.07.2007 DK 200701011; 09.07.2007 US 929665 P
(43) Date of publication of application: 17.03.2010
(73) Proprietor: Coloplast A/S, 3050 Humlebæk (DK)
(72) Inventor: TANGHOEJ, Allan, DK-2980 Kokkedal (DK)
(86) International application number: PCT/DK2008/050173
(87) International publication number: WO 2009/006906

(56) References cited:
- GB-A- 216 402
- US-A- 2 304 708
- US-A- 3 993 070
- US-A1- 2002 019 613

## Description

### FIELD OF THE INVENTION

The present invention relates to a strap mounted on a liquid reservoir, e.g. a two-compartment bag used in anal irrigation systems.

### BACKGROUND

Anal irrigation is often used to stimulate peristaltic of the intestines and thus reduce constipation. Especially paralyzed persons, e.g. suffering from spinal cord injuries, spinal bifida or multiple sclerosis suffer from decreased peristaltic function and thereby reduced function of the bowel system.

Systems for anal irrigation have been made more compact and mobile users have become more independent and are often able to perform the procedure by themselves. Many previous systems have been designed to be used by a caregiver, e.g. a nurse, who has normal dexterity, however, as many users of anal irrigation suffer from decreased dexterity, it is desirable to provide user friendly products in order to improve the quality of the users daily lives.

US3993070 describes an expandable syringe having an expandable bag with a filling means at one end thereof, and a dispensing means at the opposite end thereof. The filling means end of the syringe may be provided with a strap for suspending the syringe from a stand or other suitable support.

### SUMMARY OF THE INVENTION

The present invention relates to a reservoir bag for use in anal irrigation, which reservoir bag contains a liquid, the reservoir bag defines at least a first compartment for containing a liquid and the reservoir bag further comprises a liquid inlet and a liquid outlet allowing liquid communication with the first compartment. The liquid inlet is provided at one end of the bag and a strap is attached to the bag at a first attachment point in an area near the liquid inlet and attached to the bag at a second attachment point.

Thus, the strap facilitates handling of the bag, while it is being filled with liquid, such as water, as it is possible to support the bag close to the inlet and thereby stabilize this area. Furthermore, the strap facilitates handling of the bag in general, while also allowing a user to hang the bag in different places by the strap.

By an area near the inlet is meant within the close vicinity of the inlet that is within a radius of approximately 5 centimeters from the inlet.

The strap may be attached at a second attachment point placed in a direction across the bag from the liquid inlet. This configuration assists the user in holding the bag, as the user may enter a hand between a wall of the reservoir bag and the strap, thereby supporting the bag by the strap extending across the outside surface of the hand. The end of the bag incorporating the liquid inlet is defined as the top of the bag and the other end as the bottom of the bag. By across the bag from the liquid inlet is meant near the bottom of the bag in the longitudinal direction defined by the top and the bottom. The strap may also be attached at a second attachment point coincident with the first attachment point. Such a configuration makes it particularly easy to hang the bag on the shoulder.

For use in anal irrigation systems, the bag contains water or a saline solution, which is used to irrigate the bowels of a user. Such reservoir bags comprise at least a second compartment for containing a fluid and a fluid port for communicating with the at least second compartment. By expanding the second compartment, pressure will be applied upon the first compartment, whereby the liquid will be forced out and into the anal irrigation system.

Although the strap provides means, which make it easy to hang the reservoir bag on an arm rest, over a door handle or another object, the reservoir bag may further comprise a through going hole provided in the strap in an area around the first attachment point. The strap itself might be too wide or bulky to hang safely on such an element and therefore the through going hole makes it easy to hang the reservoir bag on a hook or another thin element.

To further ease the handling of the bag, the strap may provide an opening large enough to accommodate a hand. To provide such an opening, the length of the strap can exceed 12 centimeters, in particular between 20 and 30 centimeters. This allows for most types and sizes of hands to handle the bag. Furthermore such an opening allows the bag to be hung over an armrest of a chair or of a handicap toilet or any other protruding part immediately accessible to the user.

The strap can be formed in many different shapes and be made of many types of materials. In one embodiment the strap can be formed of a polymer sheet. Thus, in case the reservoir bag also is made of a polymer it is easy to weld the strap to the bag.

Furthermore, in yet another embodiment the strap can have many different dimensions. However, considering that the strap may rest on the hand of a user a comfortable width may be selected, which distributes the pressure across the width of the strap. For example the strap may have a width between 2 and 10 centimeters, preferably between 4 and 8 centimeters and in particular between 6 and 7 centimeters.

When liquid is filled into the liquid inlet it will run down to the bottom of the first compartment, however, as the volume of liquid increases the stability of the bag around the first attachment point may become compromised as the water level rises. Thus, in order to keep the center of gravity as low as possible the first compartment can be formed with a larger capacity in the area at the bottom of the bag than at the top. By stabilizing the bag in such a way, the risk that the bag unintentionally will turn around the hand or armrest is reduced.

Such larger capacity may be provided by a 'pear'-shaped bag, wherein the liquid inlet is provided at the narrow end of the bag allowing for a larger volume of liquid to be stored close to the bottom. This provides a center of gravity in the lower half of the reservoir bag.

The stability of the reservoir bag may alternatively or additionally be improved if the extent of the bag in a longitudinal direction from the top to the bottom is greater than the extent of the bag transverse to the longitudinal direction. The extent of the bag in a longitudinal direction corresponds to the length of the bag from the top to the bottom when the bag is empty. Likewise, the extent of the bag transverse to the longitudinal direction corresponds to the width of the bag from one side to another when the bag is empty.

In one embodiment a shoulder loop may be attachable to the strap in the area around the first attachment point. Thus, if there is no suitable place to hang the bag by use of the strap, a user may hang the reservoir bag around his or her shoulder.

As mentioned previously, the reservoir bag may be used as a reservoir for water or saline solution for use in an anal irrigation system. Thus, as described herein there is also disclosed an irrigation system for irrigation of a body cavity, the system comprising: a reservoir bag as described above, an insertion member defining an insertion end for insertion into a body cavity through a body opening of a human being, the insertion member defining at least one opening; and a liquid tube fluidly connecting the liquid outlet of the reservoir bag with the insertion member.

Furthermore, the use of a reservoir bag as described herein for anal irrigation is disclosed. In use the reservoir bag may be supported between the distal wall and the strap while liquid is poured into the liquid inlet.

### BRIEF DESCRIPTION OF THE DRAWINGS

The invention will be discussed further herein with reference to the following example embodiments, wherein
Fig. 1 illustrates a reservoir bag as disclosed herein from the rear (distal) side,
Fig. 2 illustrates a reservoir bag as disclosed herein from the front (proximal) side,
Fig. 3 illustrates a reservoir bag as disclosed herein in section along lines III-III in Fig. 1 and Fig. 2,
Fig. 4 illustrates another configuration of the strap at the reservoir bag and
Fig. 5 illustrates a reservoir bag as disclosed herein wherein a user is handling it.

### DETAILED DESCRIPTION

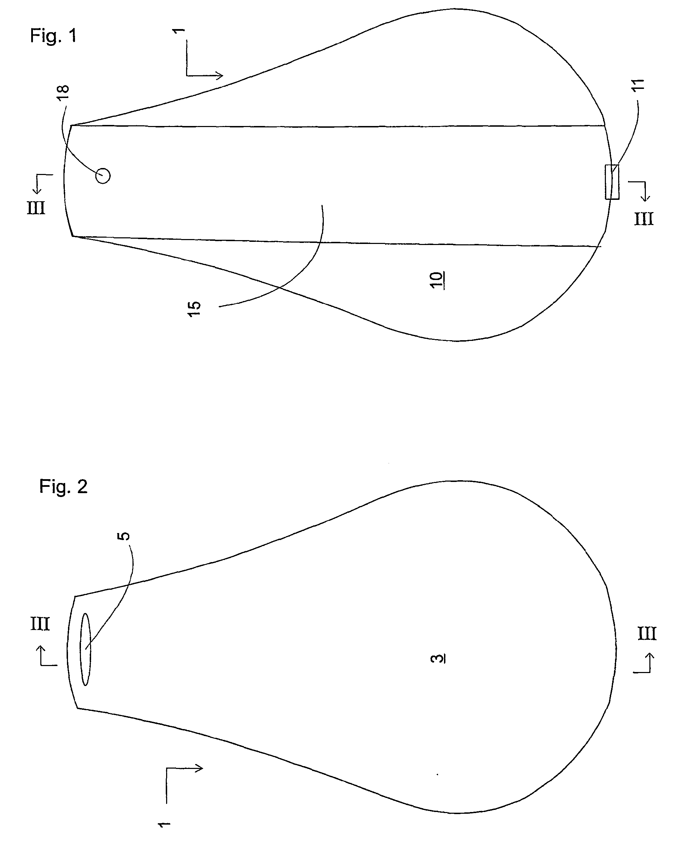

Figures 1 - 3 show an embodiment of a reservoir bag 1. The reservoir bag is adapted to contain a liquid in a first compartment 2, which is defined by a proximal wall 3 and a partition wall 4. A liquid inlet 5 in the shape of a slit in the proximal wall is provided in an upper half 6 of the reservoir bag. A liquid outlet 7 is provided in the bottom half 8 of the reservoir bag. The liquid outlet is adapted to be either closed or connected to a tube. This can for example be done by providing an inner thread (not shown) in the liquid outlet. Thus, a plug or a tube with a corresponding outer thread can for example be screwed into the liquid outlet. It should be understood that other means for closing the liquid outlet or connecting an insertion device (not shown) thereto may be provided. As described above, such insertion device may be adapted for insertion into the rectum for anal irrigation.

A second compartment 9 for containing a fluid, e.g. air, is defined by the partition wall 4 and a distal wall 10. A fluid port 11 is provided in the bottom half of the reservoir bag. The fluid port functions both as an inlet for filling the second compartment with a fluid and as an outlet for emptying the second compartment. As described above with respect to the liquid outlet 7, the fluid port can be closed or connected to a tube.

In some embodiments, the whole anal irrigation system is delivered as a single unit where all the connections are welded, molded or otherwise preformed together. Thus, the liquid outlet may for example be welded or molded together with a tube for transporting the liquid to the insertion member. In a similar manner the fluid outlet may be welded or molded with a tube transporting a fluid, e.g. air, from a pump or similar supply.

In order to prevent liquid from running back out the liquid inlet, a one-way valve 12 may be provided in the first compartment. Such one-way valves are commonly known and can be provided in many different shapes. In the present embodiment it has been provided as two valve sheets 13, 14, which are welded to the inside of the proximal wall 3 and partition wall 4, respectively, in such a way that the surfaces of the valve sheets are in close contact, only allowing liquid to flow in one direction.

The disclosed reservoir bag is for use in an anal irrigation system and as such its primary function is well known. Such anal irrigation systems are for example known from published International Application No. WO 03/030968. From here it can be understood that the bag is filled with irrigation liquid, e.g. tap water, in the first compartment. The liquid can then be evacuated through the liquid outlet through a tubular system and a probe into the rectum for anal irrigation. Pumping air into the second compartment causes the liquid to be evacuated. This builds a pressure on the first compartment, which pushes the liquid out of the reservoir bag.

The disclosed reservoir bag is furthermore provided with a strap 15, which is attached to the bag at a first attachment point 16 in the upper half of the bag in an area surrounding the liquid inlet. The strap is also attached to the bag at a second attachment point 17 in the lower half of the bag in a direction across the bag from the liquid inlet. The strap 15 stretches across the bag from the liquid inlet 5 in a direction corresponding to the direction by which the liquid will flow into the reservoir bag 1, as it is poured through the liquid inlet 5.

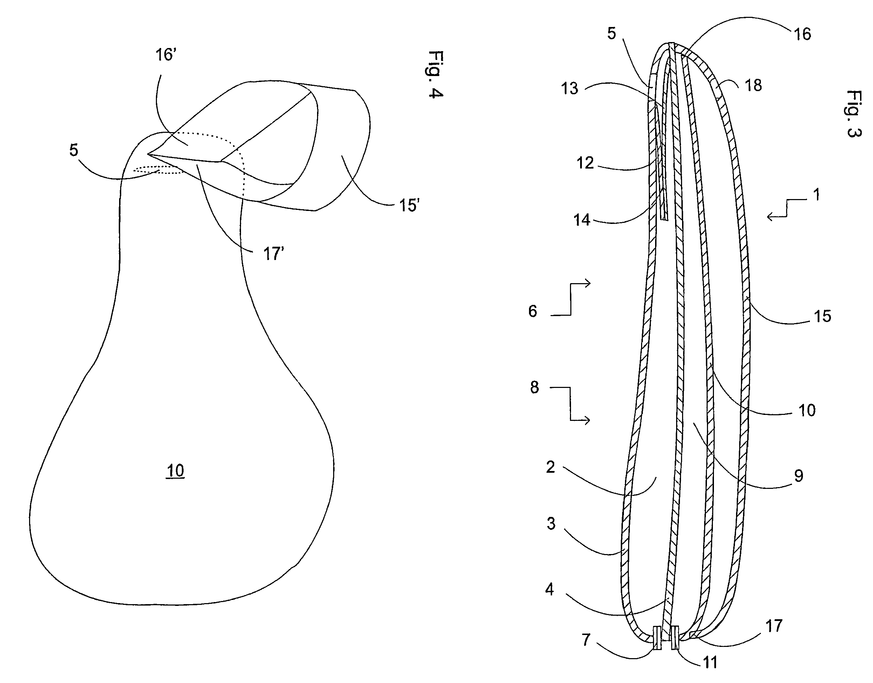

Figure 4 illustrates another configuration of a strap 15', which is attached to the bag at a first attachment point 16' and a second attachment point 17'. The first and second attachment points 16', 17', are coincident in the upper half of the bag in an area surrounding the liquid inlet.

The attachment point may be provided as welds, which can be performed at the same time as the bag is manufactured. However, the strap may also be attached to the bag at the attachment points by gluing or any other means of attachment known to the person skilled in the art.

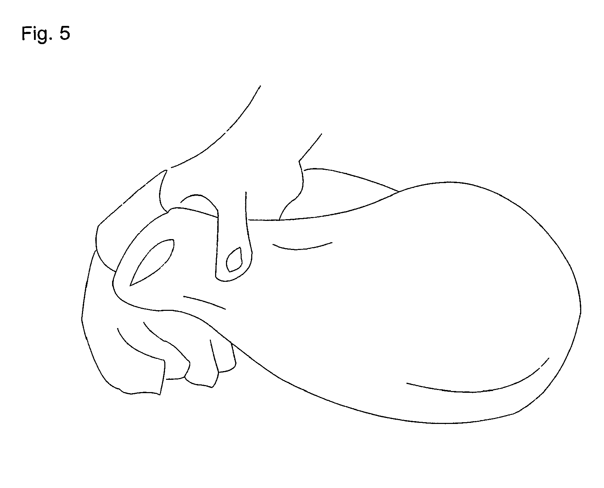

As can be seen from Fig. 5 the strap 15, 15' facilitates handling of the bag. The long broad strap is particularly user friendly for people with decreased dexterity, as their whole hand fits in the strap. As liquid is poured into the bag through the liquid inlet it will run down into the bottom half of the bag. This helps with the stability of the bag. For the embodiment of figures 1-3 it would maintain the bag in the position around the hand as shown in Fig. 5, where the reservoir bag hangs from the edge of the hand and the index finger down across the palm of the hand, while the strap extends down the outside surface of the hand. This allows the bag to rest on the edge of the hand and the index finger, however, it should be understood that the same stability might be provided if the reservoir bag is hung around any other suitable object. Furthermore, providing the major part of the first compartment in the bottom half of the bag provides a low point of gravity, which also aids in the stability of the bag. For the embodiment in figure 4, the bag would be hanged by the strap and due to the low point of gravity the bag would hang down in a stabilized position with the opening in the top. For this embodiment the user would also be able to hold the bag as illustrated in figure 5 with the hand extending through the strap and holding the bag at the upper portion (or the neck portion). In this position the strap would function as a security against dropping the bag and further the hand may carry some of the weight of the bag - as with the embodiment of figures 1-3.

As the reservoir extends down in front of the palm of a users hand, it allows the user to grab around the upper half of the reservoir bag. This further improves handling as the user can turn and twist his or her hand while still maintaining full control. In order to fit comfortably in the user's hand, the upper half of the bag may have a width between 8 and 13 centimeters in order to fit in the hand of a user. However, it should be understood that smaller or larger widths of the upper half may be provided, e.g. down to 3 or 4 centimeters and up to 18 or 20 centimeters, for different groups of people and considering different sized hands.

A through going hole 18 may also be provided in the strap in an area around the first attachment point, or in the upper half of the reservoir bag. Such hole may be used to hang the bag on a hook or similar protruding object, which may support the bag.

Although shown in the figures, the strap does not necessarily have to cross the entire extent of the bag. The second attachment point may for example be placed halfway across the surface of the distal wall in an alternative embodiment. The illustrated reservoir bag has a longitudinal extent, i.e. from the periphery of the upper half to the periphery of the lower half of 27 centimeters, where the strap has the same length. However, most hands will be able to fit into a space 12 centimeters wide, or even smaller depending on the hand. Furthermore, in order to provide comfort and prevent too much pressure on one area, the strap may also be provided with a substantial width. For example in the embodiment shown, the strap has a width of 6 centimeters which helps distribute pressure across the edge of the hand when holding the bag. However, the width of the strap may be smaller or larger depending on the size of the bag.

The bag 1 and strap 15, 15' can be formed of many different materials. However, a polymer material is preferred as this provides a wide selection of flexible and durable materials. Such polymer materials may for example be polyethylene (PE) or polypropylene (PP).

Although not shown the strap may also be provided with a shoulder loop which is large enough to arrange around a user's shoulder. Such a loop can be attached to the strap in an area in the upper half of the bag and may be either fixedly or releasable attached, for example with a snap lock or a releasable coupling. Thus, if there is no object to hang the strap onto, the user may hang the bag on his or her shoulder.

## Claims

1. A reservoir bag (1) for use in anal irrigation systems, which reservoir bag is adapted to contain a liquid, the reservoir bag defines at least a first compartment (2) for containing a liquid and the reservoir bag further comprises a liquid inlet (5) and a liquid outlet (7) allowing for liquid communication with the first compartment, wherein the liquid inlet is provided at one end of the bag and a strap is attached to the bag at a first attachment point (16) in an area near the liquid inlet and attached to the bag at a second attachment point **characterised in that** the second attachment point (17) is placed in a direction across the bag from the liquid inlet or the second attachment point (17') is coincident with the first attachment point.

2. A reservoir bag according to claim 1, wherein the reservoir bag comprises at least a second compartment for containing a fluid and a fluid port for communicating with the at least second compartment.

3. A reservoir bag according to any of the preceding claims, wherein a through going hole is provided in the strap in an area around the first attachment point.

4. A reservoir bag according to any of the preceding claims, wherein the length of the strap exceeds 12 centimeters, in particular between 20 and 30 centimeters.

5. A reservoir bag according to any of the preceding claims, wherein the strap is formed of a polymer sheet.

6. A reservoir bag according to any of the preceding claims, wherein the strap has a width between 2 and 10 centimeters, preferably between 4 and 8 centimeter and in particular between 6 and 7 centimeters.

7. A reservoir bag according to any of the preceding claims, wherein the first compartment has a larger capacity in the area at the bottom of the bag than at the top.

8. A reservoir bag according to any of the preceding claims, wherein the extent of the bag in a longitudinal direction is greater than the extent of the bag transverse to the longitudinal direction.

9. A reservoir bag according to any of the preceding claims, wherein a shoulder loop is attachable to the strap in the area around the first attachment point.

10. An irrigation system for irrigation of a body cavity, the system comprising:
- a reservoir bag according to claims 1 - 9,
- an insertion member defining an insertion end for insertion into a body cavity through a body opening of a human being, the insertion member defining at least one opening; and
- a liquid tube fluidly connecting the liquid outlet of the reservoir bag with the insertion member.

11. Use of a reservoir bag according to any of the claims 1 - 9 for anal irrigation.

12. Use according to claim 11, comprising the steps of,
- supporting the reservoir bag between the distal wall and the strap and
- pour liquid into the liquid inlet.

## Patentansprüche

1. Behälter-Beutel (1) zur Verwendung in Systemen zur Anal-Irrigation, wobei der Behälter-Beutel dazu geeignet ist, eine Flüssigkeit aufzunehmen, mindestens ein erstes Compartment (2) zur Aufnahme einer Flüssigkeit vorgibt und ferner einen Flüssigkeits-Einlass (5) und einen Flüssigkeits-Auslass (7) aufweist, die eine Flüssigkeitsverbindung mit dem ersten Compartment ermöglichen, wobei der Flüssigkeits-Einlass an einem Ende des Beutels vorgesehen ist und eine Schlaufe an einem ersten Befestigungspunkt (16) am Beutel in einem Bereich in der Nähe des Flüssigkeits-Einlasses und an einem zweiten Befestigungspunkt am Beutel angebracht ist,
**dadurch gekennzeichnet, dass**
der zweite Befestigungspunkt (17) in einer Richtung vom Flüssigkeits-Einlass über den Beutel hinweg angeordnet ist oder der zweite Befestigungspunkt (17') mit dem ersten Befestigungspunkt zusammenfällt.

2. Behälter-Beutel nach Anspruch 1, wobei der Behälter-Beutel mindestens ein zweites Compartment zur Aufnahme eines Fluids sowie einen Fluid-Anschluss zur Verbindung mit dem mindestens einen Compartment aufweist.

3. Behälter-Beutel nach einem der vorhergehenden Ansprüche, bei dem ein durchgehendes Loch in der Schlaufe in einem Bereich um den ersten Befestigungspunkt vorgesehen ist.

4. Behälter-Beutel nach einem der vorhergehenden Ansprüche, bei dem die Länge der Schlaufe mehr als 12 Zentimeter und insbesondere 20 bis 30 cm beträgt.

5. Behälter-Beutel nach einem der vorhergehenden Ansprüche, bei dem die Schlaufe aus einer Polymerfolie hergestellt ist.

6. Behälter-Beutel nach einem der vorhergehenden Ansprüche, bei dem die Schlaufe eine Breite von 2 bis 10 Zentimeter, vorzugsweise von 4 bis 8 Zentimeter und insbesondere von 6 bis 7 Zentimeter aufweist.

7. Behälter-Beutel nach einem der vorhergehenden Ansprüche, bei dem das erste Compartment in dem Bereich am Boden des Beutels ein größeres Fassungsvermögen aufweist als im oberen Bereich.

8. Behälter-Beutel nach einem der vorhergehenden Ansprüche, bei dem das Maß des Beutels in einer Längsrichtung größer ist als das Maß des Beutels quer zur Längrichtung.

9. Behälter-Beutel nach einem der vorhergehenden Ansprüche, bei dem eine Schulterschlinge im Bereich um den ersten Befestigungspunkt an der Schlaufe anbringbar ist.

10. Irrigationssystem zur Irrigation eines Körperhohlraums, wobei das System aufweist:
- einen Behälter-Beutel nach den Ansprüchen 1 bis 9,
- ein Einfüzrungsteil, das ein Einführungsende zur Einführung in einen Körperhohlraum durch eine Körperöffnung eines Menschen vorgibt, wobei das Einführungsteil mindestens eine Öffnung vorgibt,
und
- einen Flüssigkeitsschlauch, über den der Flüssigkeits-Auslass des Behälter-Beutels mit dem Einführungsteil in Flüssigkeitsverbindung steht.

11. Verwendung eines Behälter-Beutels nach einem der Ansprüche 1 bis 9 zur Anal-Irrigation.

12. Verwendung nach Anspruch 11, die folgende Schritte umfasst:
- Haltern des Behälter-Beutels zwischen der distalen Wand und der Schlaufe
und
- Einfüllen von Flüssigkeit in den Flüssigkeits-Einlass.

## Revendications

1. Réservoir (1) destiné à être utilisé dans des systèmes d'irrigation anale, lequel réservoir est adapté pour contenir un liquide, le réservoir comporte au moins un premier compartiment (2) destiné à contenir un liquide et le réservoir comprend en outre une entrée pour le liquide (5) et une sortie pour le liquide (7) permettant d'établir une communication pour le liquide avec le premier compartiment, dans lequel l'entrée de liquide est prévue à une extrémité du réservoir et une sangle est fixée au réservoir au niveau d'un premier point de fixation (16) dans une zone proche de l'entrée de liquide et est fixée au réservoir au niveau d'un second point de fixation **caractérisé en ce que** le second point de fixation (17) se trouve situé dans une direction transversale du sachet par rapport à l'entrée de liquide ou le second point de fixation (17) coïncide avec le premier point de fixation.

2. Réservoir selon la revendication 1, dans lequel le réservoir comprend au moins un second compartiment destiné à contenir un fluide et un orifice prévu pur le fluide permettant de communiquer avec ledit second compartiment.

3. Réservoir selon l'une quelconque des revendications précédentes, dans lequel un trou traversant est fourni dans la sangle dans une zone située autour du premier point de fixation.

4. Réservoir selon l'une quelconque des revendications précédentes, dans lequel la longueur de la sangle dépasse 12 centimètres et est comprise idéalement entre 20 et 30 centimètres.

5. Réservoir selon l'une quelconque des revendications précédentes, dans lequel la sangle est constituée d'une feuille en polymère.

6. Réservoir selon l'une quelconque des revendications précédentes, dans lequel la sangle présente une largeur comprise entre 2 et 10 centimètres, de préférence entre 4 et 8 centimètres et idéalement entre 6 et 7 centimètres.

7. Réservoir selon l'une quelconque des revendications précédentes, dans lequel le premier compartiment a une capacité plus importante dans la zone située au fond du réservoir que dans la zone située en haut du réservoir.

8. Réservoir selon l'une quelconque des revendications précédentes, dans lequel le développement du sachet dans une direction longitudinale est plus important que le développement du sachet transversal par rapport à la direction longitudinale.

9. Réservoir selon l'une quelconque des revendications précédentes, dans lequel une bretelle pour épaule peut être fixée à la sangle dans la zone située autour du premier point de fixation.

10. Système d'irrigation destiné à irriguer une cavité du corps, le système comprenant :
- un réservoir selon les revendications 1 à 9,
- un élément d'insertion définissant une extrémité d'insertion destinée à être insérée dans une cavité du corps à travers un orifice du corps d'un être humain, l'élément d'insertion comportant au moins une ouverture ; et
- un tube de liquide établissant une communication fluidique entre la sortie de liquide du réservoir et l'élément d'insertion.

11. Utilisation d'un réservoir selon l'une quelconque des revendications 1 à 9 pour irrigation anale.

12. Utilisation selon la revendication 11, comprenant les étapes qui consistent à :
- supporter le réservoir entre la paroi distale et la sangle et
- verser le liquide dans l'entrée prévue pour le liquide.
